# EUROPEAN PATENT APPLICATION

(11) **EP 2 441 439 A1**
(43) Date of publication of application: **18.04.2012**
(21) Application number: 11185462.6
(22) Date of filing: 17.10.2011
(51) Int. Cl.: A61K 9/00, A61K 47/02, A61K 47/10, A61K 8/00, A61K 36/00

(54) **Pharmacological aid for prevention of incipient carious lesion and dentinal hypersensibility**

(30) Priority: 18.10.2010 IT MC20100100
(71) Applicant: Straccia, Luigina, 63023 Fermo (FM) (IT)
(72) Inventor: Straccia, Luigina, 63023 Fermo (FM) (IT)
(74) Representative: Cutropia, Gianluigi

(57) **Abstract**

A pharmacological aid for prevention of incipient carious lesion and dentinal hypersensibility is disclosed, wherein said aid is a spray containing a liquid aqueous solution suitable to be sprayed in the patient's mouth. The aqueous solution comprises the following excipients: calendula, fluorides, hydroxylapatatite and thermo-gel agent suitable to transform said aqueous solution in gel, when the aqueous solution comes in contact with the patient's gums at a temperature of about 37 °C.

## Description

The present patent application for industrial invention relates to a pharmacological aid for prevention of incipient carious lesion and dentinal hypersensibility.

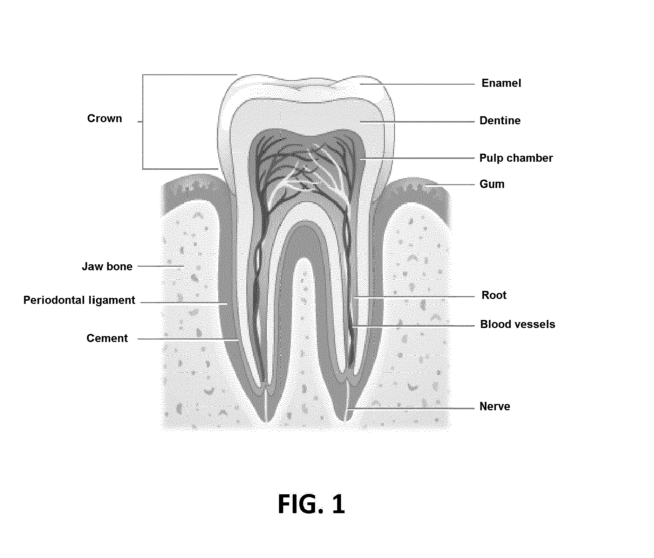

Fig. 1 is a diagrammatic sectional view of tooth and portion of mandibulary bone and gum.

Referring to Fig. 1, the tooth is made of three layers:
- external layer (enamel), which is the external, more resistant layer,
- intermediate layer (dentine), which is the softest dental substance,
- internal layer (pulp), where blood vessels and nervous terminations are situated.

Dentine has a honeycomb structure with small channels (tubules) that connect dental surface with pulp. Pulp is connected to the root, which is completely immersed in the bone, which is in turn surrounded and wrapped by gingival tissue (coating tissue).

These structures mutually interact and are extremely important for support and functionality of the dental element.

The carious lesion is a pathological destructive process of hard tissues of the tooth and is revealed by demineralization and infection of these tissues with formation of cavities extending from the periphery of the tooth towards the pulp. The formation of cavity is revealed by decalcification of enamel and destruction of dentine; infection also involves the pulp with inflammation and consequent necrosis. The carious process affects: grooves, slots, blind foramina, interproximal surfaces, and cervical areas.

Incipient carious lesion (enamel caries) is characterized by a change in the color and transparency of the tooth; it is revealed by a white-chalky stain; the enamel surface is eroded and irregular. Such change can be noticed with the tip of a probe.

Upon histological examination enamel caries is characterized by gradual decalcification of enamel prisms, which start disintegration, leading to complete destruction.

The enamel lesion has dark translucent areas with morphological alteration of prisms. Then prisms are demineralized and disintegrated because of the activity of acidophilic agents (acid substances produced by bacterial plaque). Decalcification extends deeply according to the direction of prism beams, with layers extending in radial direction towards the surface of dentine.

Caries is formed in a small enamel imperfection, is revealed in the point where three o more lobes of tooth during growth are joined and in the imperfect closing of the laminae of the enamel (occlusar grooves of molars and premolars); it occurs at the end of tooth grooves (vestibular groove of molars).

Caries is also formed in smooth surfaces where no cavities, no grooves or no other imperfections are present and occurs in regions where bacterial plaque is collected, such as interproximal surfaces of teeth, cervical third of teeth and other regions that are difficult to clean.

Dentinal hypersensibility represents a physiological sign of vitality and at the same time is a valid defense mechanism of the tooth against harmful external stimuli; it is basically related with gingival retraction phenomena, enamel loss and wear of radicular cement and is characterized by symptoms from simple trouble to acute pain, due to exposure of dentine to thermal, chemical, mechanical, osmotic, and tactile stimuli. In many cases dentinal hypersensibility has little duration and disappears with elimination of stimulus; it is occasionally more intense and persists also after eliminating the stimulus.

It is important to identify the causes of dentinal hypersensibility through a correct diagnosis with objective examination and x-rays of affected teeth. The pain perceived by the patient must be differentiated from a whole series of other symptoms, which require specialized treatments, such as carious lesions, fractured teeth, infiltrated restorations, occlusal trauma, post-surgery hypersensibility. In order to get to a certain diagnosis, other possible causes of dental pain must be excluded.

Hypersensibility affects one fourth of population in age range from 20 to 40 years, in higher extent for females. Such a pathology can affect all dental elements, although it mostly affects cervico-vestibular surfaces of incisors, canines and premolars. Pain occurs when dentine is exposed, and may be related both to enamel loss and apical migration of gingival margin, due to recession or paradontal surgery therapy.

Painful symptomatology due to dentine exposure to external stimuli of physical (hot-cold), chemical (acid-sweet), mechanical (brushing, probing) type is determined according to the hydrodynamic theory by the movement of dentinal fluid contained in dentinal tubules. Such a movement causes a deformation of odontoblastic prolongations (Tomes fibers), which affects nervous terminations, activating them.

Stimuli, such as air jets, cause a movement of the tubular fluid towards the pulp and its nervous fibers, causing a painful stimulus. The contact with cold substances causes a contraction of the fluid, whereas the hot stimulus produces a centripetal movement towards the pulpo-dentinal end. The pain caused by a hot stimulus is dull, whereas the pain caused by a cold stimulus is acute.

Modern literature indicates that evoked pain can be of two types and hot stimuli require a longer time to occur with respect to the one caused by cold stimuli.

According to the hydrodynamic theory, therapies are currently aimed at maintaining the stability of dentinal fluid, externally sealing dentinal tubules with application of resins or lacquers, or reducing the diameter of tubules with the use of chemical-topical agents. These actions can reduce pain; in fact, the purpose is to make the dental flow stable or to close tubules and therefore isolate nervous terminations from any irritative and traumatic stimulus.

The therapeutic approach for treatment of dentinal hypersensibility is related with the lesion of affected dental elements. If it is not necessary to restore the morphology of the dental element, the operating protocol provides for using chemical-topical substances that, by reacting with the structural components of the tubules, form insoluble compounds able to obstruct the dentinal tubule; or for using lacquers or resins that occlude the lumen of the dentinal tubule.

The chemical-topical substances are Fluorides, Oxalates, Hydroxylapatite, Calcium Hydroxide, Insoluble Salts (Ca- Sa-K).

Fluorides are: sodium fluoride, stannous fluoride, sodium monofluorophosphate. They react with calcium of the dentinal fluid and form fluorapatite or calcium fluoride.

Clinical investigations on dentinal hypersensibility have shown that treatments with fluorinated toothpaste and concentrated fluoride solutions are very effective. Lukomsky was among the first ones to suggest topical use of sodium fluoride (NaF) for treatment of dentinal hypersensibility, demonstrating that in vitro such a compound reduces the movement of dentinal fluid by approximately 18%. The action mechanism of sodium fluoride determines a reduction of the diameter of the tubules, and permits precipitation of calcium fluoride with substitution of fluorapatite to hydroxyapatite.

Different methods are used for application of fluorides, such as for example fluorinate solutions (gel, adhesive paste), fluorinated paints, fluorinated toothpaste. These solutions are put in contact with enamel by means of brushing, mouthrinse, or ionophoresis. These products employ strontium chloride at 10% or basic sodium citrate in peluronic gel at 2%, or potassium nitrate at 5%.

The use of chemical substances in the form of mouthrinse removes the plaque or at least facilitates mechanical removal. The mouthrinse must have some requisites, such as absence of toxicity, action stability, incapability of inducing resistant microbic populations, capability of removing the plaque and inhibit its formation. Its use is suggested both before and after the use of toothbrush, since it acts in mechanical removal of the plaque and inhibits its formation.

The main substances used in the composition of a mouthrinse are antiseptic (clorexidina, sanguinarina, fluorides), anti-plaque enzymes and some plaque modifier agents.

The action mechanism of clorexidina is based on the capability of reducing adhesion and bacterial cohesion. The current dose of clorexidina is 0.1-0.2%. If used for long, it may create dark pigmentations on dental surfaces and oral mucosae, and cause taste alterations.

Sanguinarina interferes on the adhesion mechanism of bacteria in plaque formation. It produces pharmacological effects at 0.1-0.2% concentrations.

Fluorine is amongst the most used chemical agents; it is the base element in caries prevention. Its action is expressed through the reduction of solubility of crystals of the enamel, remineralization and antienzymatic action. The use of fluorinated mouthrinses does not determine toxic effects; in general, preparations contain fluorine in the amount of 0.5% at ph comprised between 2.5 and 4.

Chemical substances are known, such as oxalates (ferric nitrate, potassium nitrate, potassium oxalate), able to reduce the diameter of dentinal tubules. Numerous scientific works both in vitro and in vivo have demonstrated the good efficacy of these treatments. Said substances form a layer of insoluble acid-resistant crystals inside dentinal tubules, which reduce the gauge of tubules, and consequently the permeability of dentine, almost to zero. Clinical studies (Muzzin and Johnson1989; Yeh 1990) have especially confirmed the efficacy of potassium oxalate and ferric oxalate, which are capable of eliminating dentinal sensibility also in very severe cases.

Among the different substances used for dentinal hypersensibility, the most effective ones determine the direct occlusion of dentinal tubules and the choice is currently based on the use of toothpaste that contain desensibilization substances (Addy 1983; Barone 1991; Dowell 1983).

Various studies have demonstrated the efficacy of hydroxylapatite (Ca10 (P04)6 (OH)2 ), which is the main inorganic constituent of the hard tissues of teeth. This compound acts through occlusion of exposed dentinal tubules.

Wannenmarcher has proposed the use of hydroxilapatite in toothpaste to seal dentinal tubules, polish and smooth dental surface, and improve efficacy of oral hygiene. According to some authors, topical application of hydroxylapatite would also have a cariostatic effect for inhibition of plaque formation and remineralization of carious lesion. Gel at 15 % ultramicronized hydroxylapatite contains more than 50% of hydroxylapatite microcrystals with 0.2-1.00 micron diameter. Once applied, the gel provides good adhesion of medication on tooth; said compound is very effective and has no side effects.

Another method to isolate exposed dentine is the use of calcium hydroxide, frequently used as cavity substratum. Its action consists in stimulating the production of peritubular (secondary) dentine. For many years calcium hydroxide has been used to treat dental hypersensibility, especially after root planing. Mjor and Furseth (1968) have demonstrated that calcium hydroxide determines the formation of dentine from reaction.

Recently, insoluble calcium and strontium salts and soluble potassium salts have been used to treat dental hypersensibility to obtain a desensibilizing substance composed of two solutions: the first compound is represented by the association of two soluble potassium salts.

Said compounds intervene with double action mechanism based on precipitation of insoluble salts occluding tubular orifices, and on simultaneous formation of a soluble potassium salt that depolarizes nervous fibers, enhancing the sensibility threshold.

In the clinical practice, in addition to clinical solutions determining dental hypersensibility as illustrated above, these solutions can be applied also for patients with deep caries in replacement of substratum, patients with stumps prepared for prosthetic purposes, patients with preparations for inlays and bruxist patients.

A therapy of dentinal hypersensibility for professional use is the tubule sealing technique with resins o lacquers, which are able to occlude the mouth of the tubules offering a good desensibilization. This technique consists in mordanting the dentine surface to enlarge the mouth of the channels and is dried with air jet and finally resin is applied. The only defect is time duration: these resins suffer from wear and must be reapplied after a period of 3/6 months.

It must be considered that dental pulp has some natural defenses that protect it from irritative stimuli. Among them, pain, inflammation, formation of secondary dentine. Moreover, dentine seems to desensibilize in a totally spontaneous way, thanks to substances coming from saliva and plasma, to plaque components and bacteria. In such a case, a mineralized barrier similar to tartar consistency is formed. Such a barrier obstructs the mouth of exposed tubules, and consequently transmission of painful stimuli.

As illustrated above, the causes of dental hypersensibility are: exposure, abrasion and mechanical wear of dentine, and apical migration of gingival margin, accentuated by presence of bacterial plaque, predisposing factors, frequent assumption of cariogenetic good, decalcification and demineralization of tooth enamel.

The classical therapeutic approach for prevention of dental hypersensibility provides for the use of fluorine toothpaste and disinfectant mouthrinses (fluorines, clorexidina, insoluble salts) and lacquers or resins for professional use.

However, said therapeutic products are impaired by some drawbacks. Lacquers or resins require application by a dentist.

Toothpaste and mouthrinse are not very effective, because of short permanence on dental structures. Such a permanence is not sufficient for the action of active principles to be absorbed by the dental structure and therefore to act.

WO2004/069278; WO02/41837; WO03/037276 patent documents disclose viscosity modifiers, such as Polaxamer and Pluronic, used in oral cavity together with anti-inflammatory substances.

W02005/063184 discloses the use of plant extracts, in particular calendula, for treatment of oral disease, such as gingivitis, plaque and tartar.

The purpose of the present invention is to eliminate the drawbacks of the prior art, providing a pharmacological aid for prevention of incipient carious lesion and dentinal hypersensibility, which is efficient, efficacious and easy to be self-administered by the patient.

This purpose has been achieved according to the invention, with characteristics claimed in independent claim 1.

Advantageous embodiments appear from the dependent claims.

According to the invention, the pharmacological aid for prevention of incipient carious lesion and dentinal hypersensibility consists in a spray containing a liquid aqueous solution suitable to be sprayed in the patient's mouth. Said aqueous solution comprises the following excipients dissolved in water:
- calendula,
- fluorides,
- hydroxylapatite, and
- thermo-gel agent suitable to convert said aqueous solution in gel, when the aqueous solution comes in contact with the patient's gums at a temperature of about 37 °C.

An essential excipient of the composition is calendula. Advantageously, Calendula is used in tincture comprising about 30 - 50% of calendula triturated leaves and alcohol for the remaining part. Advantageously, said tincture comprises 60% of ethanol at 96° and 40% of calendula leaves.

The quantity of calendula tincture is comprised between 8 - 15% with respect to the total weight of solution.

The quantity of fluorides is comprised between 0.03 - 0.15% with respect to the total weight of solution.

The quantity of hydroxylapatite is comprised between 12 - 18 % with respect to the total weight of solution.

The quantity of thermo-gel agent is comprised between 15 - 20% with respect to the total weight of solution.

In the Official Pharmacopoeia, the section on "herbs and plants that can be used by chemists in preparation of health products" includes Calendula, composed of dried flowers, either entire or cut, containing not less than 0.4% of flavonoids; the material complies with monograph of European Pharmacopoeia and refers to dry drug.

Calendula has the following chemical composition:
1) Triterpene saponins
2) Free esterified triterpene alcohols
3) Carotenoids
4) Flavonoids
5) Polysaccharides
6) Sterols
7) Sesquiterpenoids
8) Essential oil

Experiments in vitro have demonstrated antimicrobic and antiviral, antioxidant, angiogenic, anti-inflammatory effects.

Experiments in vivo have demonstrated effects on wound healing. Calendula extract favors wound healing by means of two mechanisms; it stimulates the activity of fibroblasts and migrant cells and significantly increases angiogenesis at cutaneous level.

Calendula contains hydrosoluble compounds capable of favoring hyalurinane deposition and increase tissue neovascularization, with consequent anti-inflammatory, anti-edematous and anti-tumoral effects.

Toxicity and secondary effects of calendula include sensitivity to members of Compositae family and weak cutaneous sensibilization, but no cases of dermatitis from contact are documented.

The combination of calendula consists in a double action mechanism:
- de-sensibilizing effect that reduces the degree of dental sensitivity and simultaneously pain intensity; and
- anti-inflammatory and disinfectant effect that provides anti-microbic action for inflammatory disease of periodontium.

Amongst fluorines, sodium monofluorophospate and/or sodium flourine are preferably used.

Advantageously, a potassium salt is used as active substance of fluorines, preferably potassium sorbate. Moreover, potassium sorbate has a preservation and sterilization action, killing microbes in solution water. The quantity of potassium salts in weight percentage is comprised between 0.1 - 0.3% of aqueous solution.

Preferably, a gel of hydroxylapatite ultra-micronized at 15% is used.

As thermo-gels, preferably Poloxamer® is used, and in particular Poloxamer 407® (CAS 9003-11-6 ). Such a type of thermo-gel is disclosed in patent US4,474,753. Alternatively, Carbopol ® 980 can be used as thermo-gel agent. The quantity of thermo-gel in weight percentage is comprised between 15-20% with respect to the total weight of solution. With such a quantity of thermo-gel, when the aqueous solution comes in contact with the patient's mouth at a temperature of approximately 36-37°C, it increases the viscosity of the aqueous solution, which is transformed in gel, and guarantees a permanence time of gel on tooth enamel of about 20 minutes.

The action of Poloxamer® is certainly decisive to guarantee adhesion of gel to enamel. Permanence time of 20 minutes of gel on tooth enamel allows calendula, fluoride and hydroxylapatite to create an important chemical-physical bond for the prevention and treatment of incipient carious lesion (enamel caries) and dentinal hypersensibility.

Enamel remineralization occurs in this phase, that is transformation of hydroxilapatite into fluorapatite, with consequent reduction of enamel solubility, precipitation of insoluble salts simultaneously with occlusion of dentinal tubules, and occlusion of tubules allows for reducing and eliminating painful symptomatology caused by dentinal hypersensibility, inhibition of cuticola formation and its chemiotactic action.

Advantageously, in the composition according to the invention, aromatizers can be added in a quantity comprised between 0.3-0.7% with respect to the total weight of the composition. The use of aromatizers ensures compliance of patient, with good product taste and mouth sensation.

Finally, a pH regulating substance is added to the aforementioned excipients until PH=5 is obtained. Since excipients are basic, citric acid can be used as PH regulator, for example in 5% solution.

Aqueous solution is prepared in a container provided with a pump or compressed air spray system. The use of the spray allows for domestic use in a simple, rapid way. The diffusion of the aqueous solution by spraying occurs rapidly and uniformly on all dental surfaces (grooves, slots, dentine and radicular cement exposure). Moreover, the initial liquid consistency of the aqueous solution allows for distributing the product also on gingival tissues, with bacteriostatic and bactericidal action.

Following is a recipe for production of 100 g of aqueous solution.

| EXCIPIENTS | 9 |
|---|---|
| calendula | 10 g |
| sodium monofluorophosphate | 0.05 g |
| sodium fluoride | 0.05 g |
| Gel of hydroxylapatite ultra-micronized at 15% | 15 g |
| poloxamer 407® | 17.5 g |
| aroma | 0.5 g |
| potassium sorbate | 0.2 g |
| 5% solution citric acid | Quantity sufficient to obtain ph=5 |
| sterile water for injectable preparations | Quantity sufficient to obtain 100g of aqueous solution |

### CLINICAL EXPERIMENTATION

Experimentation was carried out on 10 patients with signs and symptoms of dentinal hypersensibility.

### SIGNS

### - Dentine exposure

### SYM PTOMS

### - Severe sensibility to cold (acute pain)

### - Moderate sensibility to cold (dull pain)

(Light, moderate and severe indicate the sensibility level, therefore pain intensity).

### CLINIC CASE 1

A Fluoride spray (sodium sodium-fluoride monofluorophosphate) was applied to two patients, morning and evening for three days. Tissues were inspected every day for four days. After the fourth day the following symptoms were noted:
- Moderate sensibility to cold.
- Moderate sensibility to hot.

As it is known, fluorides have a bacterio-statical effect and therefore they have slightly reduced sensibility to cold symptoms. Sensibility to hot persisted.

### CLINIC CASE 2

To obtain better results, a spray with hydroxylapatite ultramicronized at 15% was used and applied to two patients, morning and evening for three days. Tissues were inspected every day for four days. After the fourth day the following symptoms were noted:
- Light sensibility to cold.
- Moderate sensibility to hot.

As it was expected, hydroxylapatite obtained better results than Fluorides in reducing sensibility to cold, because of the mechanical action of crystals in channel obstruction. However, sensibility to hot persisted.

### CLINIC CASE 3

A Fluoride-Hydroxylapatite spray was applied to two patients, morning and evening for three days. Tissues were inspected every day for four days. After the fourth day the following symptoms were noted:
- Light sensibility to cold.
- Moderate sensibility to hot.

Said test did not show any synergetic effect between Fluoride and Hydroxylapatite.

### CLINIC CASE 4

A Calendula spray was applied to two patients, morning and evening for three days. Tissues were inspected every day for four days. After the fourth day the following symptoms were noted:
- Lack of painful sensibility to cold.
- Light sensitivity to hot

These results were predictable because of the healing effect of Calendula. However, a slight sensibility to hot persisted, and in any case healing time was too long (four days).

### CLINIC CASE 5

A Calendula-Fluoride-Hydroxilapatite spray according to the invention was applied to two patients, morning and evening for three days. Tissues were inspected every day for four days. Surprisingly, after the second day, a lack of sensibility to cold and hot was noted. After the fourth day the following symptoms were noted:
- Lack of sensitivity to cold (from second day)
- Lack of sensitivity to hot (from second day)

Said tests demonstrate the synergetic effect of Calendula with Fluorides and Hydroxylapatite.

Application of fluorides and hydroxylapatite is effective to reduce sensibility to cold. The inclusion of Calendula and thermo-gel vehicle is more efficacious to eliminate from the second day painful sensibility to cold and hot.

The therapeutic action of these substances occurs mainly when they have a higher permanence time (10 minutes) on tissues, dentine and enamel. The introduction of the thermo-gel vehicle perfectly meets these requirements.

## Claims

1. Pharmacological aid for prevention of incipient carious lesion and dentinal hypersensibility, in which said aid is a spray containing a liquid aqueous solution suitable to be sprayed in the patient's mouth, said aqueous solution comprising the following excipients:
- calendula,
- fluorides,
- hydroxylapatite, and
- thermo-gel agent suitable to convert said aqueous solution in gel, when the aqueous solution comes in contact with the patient's mouth at a temperature of about 36 °C.

2. Aid as claimed in claim 1, **characterized in that** said is in tincture comprising 30 - 50% of calendula leaves and alcohol for the remaining part.

3. Aid as claimed in claim 2, **characterized in that**:
- the quantity of calendula tincture is comprised between 8 - 15% with respect to the total weight of solution,
- the quantity of fluorides is comprised between 0.03 - 0.15% with respect to the total weight of solution,
- the quantity of hydroxylapatite is comprised between 12 - 18 % with respect to the total weight of solution, and
- the quantity of thermo-gel agent is comprised between 15 - 20% with respect to the total weight of solution.

4. Aid as claimed in any one of the preceding claims, **characterized in that** said fluorides comprise sodium monofluorophosphate and/or sodium fluoride.

5. Aid as claimed in any one of the preceding claims, **characterized in that** said aqueous solution also comprises a potassium salt, comprising potassium sorbate as active substance of said fluorides.

6. Aid as claimed in claim 5, **characterized in that** the quantity of potassium salt is comprised between 0.1 - 0.3 % with respect to the total weight of solution.

7. Aid as claimed in any one of the preceding claims, **characterized in that** said thermo-gel agent comprises Poloxamer®.

8. Aid as claimed in any one of the preceding claims, **characterized in that** said hydroxylapatite is gel with ultramicronized hydroxylapatite at 15%.

9. Aid as claimed in any one of the preceding claims, **characterized in that** said aqueous solution comprises aromatic substances and citric acid until PH=5 of the solution is obtained.

10. Use of aid as claimed in any one of the preceding claims for the prevention of incipient carious lesion and dentinal hypersensibility.

11. Use according to claim 10, **characterized by** the fact that said aid is used to eliminate sensibility to hot due to carious lesion.

12. Use according to claim 10, **characterized by** the fact that said aid is used to eliminate sensibility to cold due to carious lesion.
